# EUROPÄISCHE PATENTSCHRIFT

(11) **EP 1 339 674 B1**
(45) Veröffentlichungstag und Bekanntmachung des Hinweises auf die Patenterteilung: **14.03.2007**
(21) Anmeldenummer: 01962988.0
(22) Anmeldetag: 30.08.2001
(51) Int. Cl.: C07C 271/12

(54) **POLYCARBAMATE UND VERFAHREN ZU IHRER HERSTELLUNG**
POLYCARBAMATES AND METHOD FOR THE PRODUCTION THEREOF
POLYCARBAMATES ET PROCEDE DE FABRICATION

(30) Priorität: 07.09.2000 DE 10044165
(43) Veröffentlichungstag der Anmeldung: 03.09.2003
(73) Patentinhaber: BASF Coatings AG, 48165 Münster (DE)
(72) Erfinder: HENKELMANN, Jochem, 68165 Mannheim (DE); MAAS, Heiko, 68165 Mannheim (DE); STAMM, Armin, 55268 Nieder-Olm (DE); RINK, Heinz-Peter, 48153 Münster (DE); JUNG, Werner-Alfons, 59387 Ascheberg (DE)
(74) Vertreter: Isenbruck, Günter
(86) Internationale Anmeldenummer: PCT/EP2001/010030
(87) Internationale Veröffentlichungsnummer: WO 2002/020468

(56) Entgegenhaltungen:
- EP-A- 0 594 068
- EP-A- 0 767 230

## Beschreibung

Die Erfindung betrifft aliphatische Polycarbamate sowie ein Verfahren zu ihrer Herstellung.

Aliphatische Polycarbamate, das heißt Carbamate mit mehr als einer, bevorzugt mehr als zwei Carbamatgruppen im Molekül, sind bekannt und werden unter anderem als Vemetzer zur Herstellung von Automobillacken eingesetzt. Da die Herabsetzung des Lösemittelgehalts von Lacken zur Verminderung von Emissionen angestrebt wird, ist es zur Einhaltung einer für das Aufbringen des Lacks ausreichend niedrigen Viskosität notwendig, Lackbestandteile mit niedriger Viskosität zur Verfügung zu haben.

EP-A 0 594 068 offenbart eine härtbare Zusammensetzung enthaltend ein Polycarbamat der allgemeinen Formel A 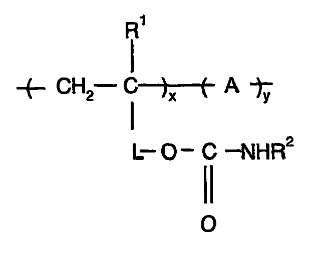 mit R¹ = H oder CH₃, R² = H, Alkyl oder Cycloalkyl, L = eine divalente verbindende Gruppe, A = eine Wiederholungseinheit, die von einem ethylenisch ungesättigten Monomeren abgeleitet ist, x = 10 - 90 Gew.%, y = 10 - 90 Gew.-% und x + y = 100 Gew.-%.

Aufgabe der Erfindung ist es, neue aliphatische Polycarbamate, die bei niedriger Viskosität mehrere Carbamat-Gruppen im Molekül enthalten und deren Molekülgröße und Anzahl an Carbamat-Gruppen sich nach Wunsch variieren läßt, sowie ein Verfahren zu ihrer Herstellung zur Verfügung zu stellen.

Erfindungsgemäß werden Polycarbamate der allgemeinen Formel I 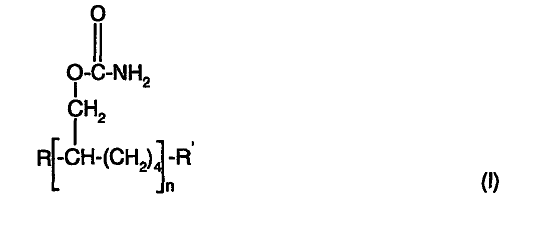 vorgeschlagen, worin
- R und R': gleiche oder verschiedene Alkylgruppen mit 1-4 C-Atomen bedeuten und
- n: eine Zahl von im Mittel 2 bis 5 ist.

Erfindungsgemäß wird ferner ein Verfahren zur Herstellung von Polycarbamaten der allgemeinen Formel I vorgeschlagen, das darin besteht, daß man einen Polyalkohol der Formel II, 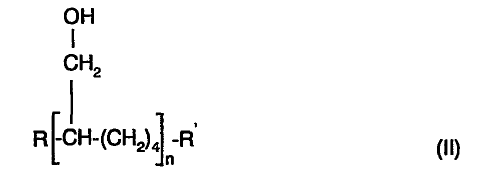 worin R, R' und n die oben angegebene Bedeutung haben mit Phosgen, vorzugsweise im Überschuß, zu einem Polychlorkohlensäureester der Formel III, umsetzt

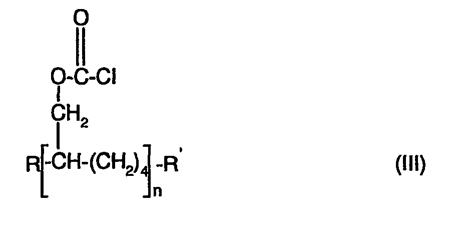

Der Polychlorkohlensäureester III wird anschließend mit Ammoniak in das Polycarbamat I überführt.

Als Polycarbamate und Polyalkohole sollen im Rahmen dieser Beschreibung solche Verbindungen verstanden werden, die - jedenfalls im Mittelwert bei Homologengemischen - mehr als 2 Carbamat- oder OH-Gruppen enthalten, das heißt im wesentlichen solche Vertreter, die als Oligomere bezeichnet werden können. Die erfindungsgemäßen Polycarbamate liegen häufig als Gemische von Homologen vor, die hauptsächlich Vertreter mit n = 1 bis etwa n = 10 enthalten.

Die Aminierung wird im allgemeinen bei einer Temperatur von 5 bis 50°C vorzugsweise bei 25 bis 30°C und bei einem Druck von 1 bis 10 bar, vorzugsweise 1 bis 3 bar durchgeführt. Dabei kann in einem inerten Lösungsmittel wie Toluol, Chlorbenzol, Heptan, Dichlorethan und Pentylacetat gearbeitet werden. Vorzugsweise wird die Aminierung der Polychlorkohlensäureester ohne Zusatz eines inerten Lösungsmittels durchgeführt.

Die Verbindungen der Formel II sind bekannt und zum Beispiel in der DE-A 196 54 167 beschrieben. Sie werden bevorzugt durch katalysierte Metathesereaktion von Cyclopenten enthaltenden Kohlenwasserstoffgemischen, zum Beispiel Erdölfraktionen, Hydroformylierung der Reaktionsprodukte und nachfolgende Umsetzung mit Wasserstoff an einem Hydrierungskatalysator erhalten.

Die Umsetzung der Polyalkohole mit Phosgen wird im allgemeinen bei einer Temperatur von + 5 bis 65°C, besonders vorteilhaft bei einer Temperatur von + 40°C bis 45°C durchgeführt. Das Phosgen wird im allgemeinen in einem Überschuß von 5 bis 20 mol-% einwirken gelassen, so daß normalerweise praktisch alle primären Hydroxygruppen zu Chlorformiatgruppen umgesetzt werden.

Die Umsetzung der Polyalkohole kann ohne Zusatz von inerten Lösungsmitteln durchgeführt werden. Im allgemeinen ist es jedoch von Vorteil, in einem aprotischen Lösemittel wie Toluol zu arbeiten.

Die erfindungsgemäßen Polycarbamate werden als Lackbestandteile, insbesondere in Automobillacken, verwendet, wo sie zur Regulierung der Viskosität zugesetzt werden.

Die folgenden Beispiele beschreiben bevorzugte Ausführungsformen der Erfindung. Die darin angegebenen OH-Zablen bedeuten g OH je 100 g Substanz. Die Bestimmung der OH-Zahl ist in DE-A 196 54 167 beschrieben.

### Beispiel 1

### Herstellung des Polychlorkohlensäureesters III

In einem 0,5 l fassenden Glasrührkolben mit Gaseinleitungsrohr und einem Solekühler von - 15°C und einem Trockeneiskühler wurden 50 g Toluol vorgelegt und bei 40-45°C 80 g Phosgen eingeleitet, bis sich Rückfluß einstellte. Dazu wurden innerhalb von 7 Stunden 150 g mehrwertiger Alkohol entsprechend der Formel II in 50 g Toluol mit der OH-Zahl von 359 und weitere 135 g Phosgen gegeben. Danach wurde 2 Stunden lang bei 40 bis 45°C nachgerührt. Anschließend wurde bei 40-45°C so lange Stickstoff durch die Reaktionsmischung geleitet, bis alles Phosgen entfernt war. Man erhielt 197 g Chlorformiat III. Der Alkoholumsatz war quantitativ.

### Beispiel 2

### Herstellung des Polycarbamats I

In einer 0,5 l Glasapparatur wurden wie in Beispiel 1 240 g Polyalkohol (OH-Zahl 359) in 50 g Toluol mit 217 g Phosgen umgesetzt. Der Polyalkohol wurde ohne Verdünnung mit einem Lösungsmittel eingesetzt. Nach 5 Stunden Reaktion bei 50°C wurde 1 Stunde mit Stickstoff phosgenfrei gestrippt. Anschließend wurde in einem 1 1-Glasrührkolben 3 h lang bei 25-30°C bis zur Sättigung Ammoniak eingeleitet. Während der Ammoniak-Einleitung bildete sich ein weißer Niederschlag. Nach Reaktionsende wurde das Produkt in 1,3 l Toluol und 250 ml Wasser gelöst. Die Wasserphase wurde abgetrennt und die organische Phase mit 250 ml Wasser gewaschen. Die organische Phase wurde anschließend an einem Rotationsverdampfer eingeengt. Man erhielt 270 g eines hellbraunen Feststoffs mit einer OH-Zahl von 13. Das IR-Spektrum zeigte die Carbamat-Bande als einzige funktionelle Gruppe.

## Patentansprüche

1. Polycarbamate der allgemeinen Formel I oder deren Homologengemische 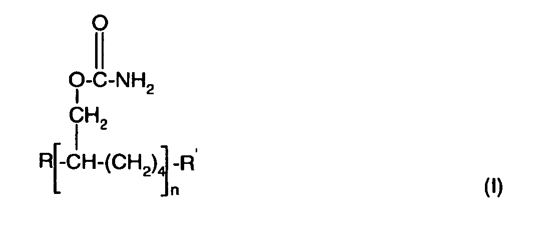 worin
R und R' gleich oder verschieden sind und Alkylgruppen mit 1-4 C-Atomen bedeuten und
n eine ganze Zahl ist, wobei der Mittelwert von ri eine Zahl von 2 bis 5 ist.

2. Polycarbamate nach Anspruch 1, **dadurch gekennzeichnet, daß** sie aus einem Gemisch von Homologen mit n = 1 bis n = 10 bestehen.

3. Verfahren zum Herstellen von Polycarbamaten der allgemeinen Formel I gemäß Anspruch 1 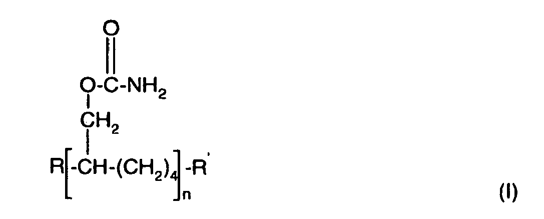 worin
R und R' gleiche oder verschiedene Alkylgruppen mit 1-4 C Atomen sind und
n eine Zahl von im Mittel 2 bis 5 ist,
**dadurch gekennzeichnet, daß** man einen Polyalkohol der Formel II 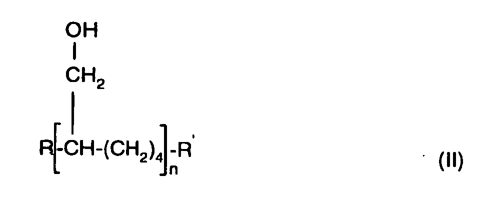 worin R, R` und n die vorstehend angegebene Bedeutung haben,
mit Phosgen zu einem Polychlorkohlensäureester der Formel III 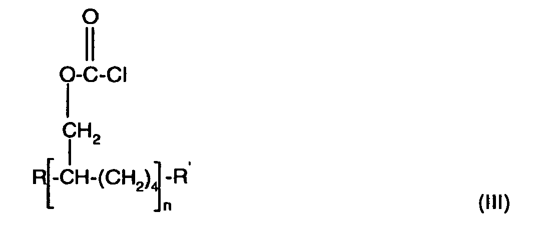 umsetzt und den Polychlorkohlensäureester III anschließend mit Ammoniak in das Polycarbamat I überführt.

4. Verfahren nach Anspruch 3, **dadurch gekennzeichnet, daß** man die Umsetzung in einem aprotischen Lösemittel durchführt.

5. Verfahren nach Anspruch 3 oder 4, **dadurch gekennzeichnet, daß** man die Umsetzung mit Phosgen bei + 5 bis + 65°C und die Umsetzung mit Ammoniak bei 5 bis 50 °C durchführt.

6. Verwendung der Polycarbamate der allgemeinen Formel I gemäß Anspruch 1 als Lackbestandteil.

7. Verwendung nach Anspruch 6 in Automobillacken.

## Claims

1. A polycarbamate of the formula I or homolog mixtures thereof 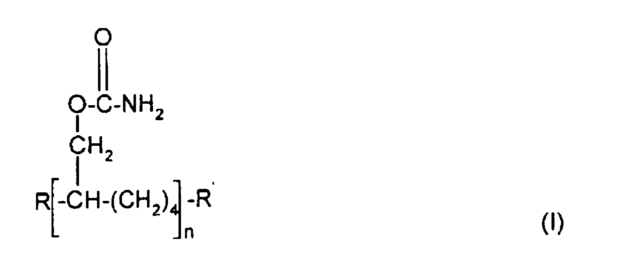 in which
R and R' are identical or different and are alkyl groups having 1-4 carbon atoms and
n is an integer, the average of n being a number from 2 to 5.

2. The polycarbamate according to claim 1, which consists of a mixture of homologs where n= 1 to n = 10.

3. A process for the preparation of polycarbamates of the formula I according to claim 1 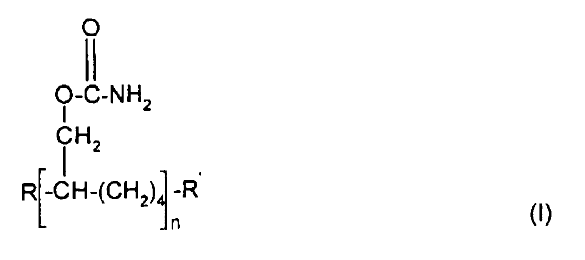 in which
R and R' are identical or different and are alkyl groups having 1-4 carbon atoms and
n is a number from, on average, 2 to 5,
which comprises reacting a polyalcohol of the formula II 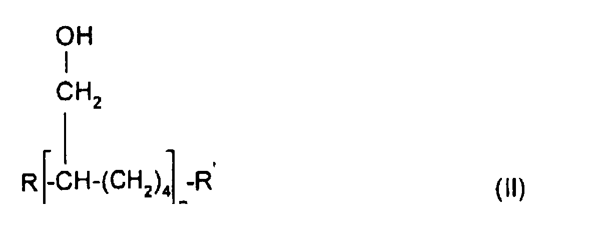 in which R, R' and n are as defined above,
with phosgene to give a polychlorocarbonic ester of the formula III 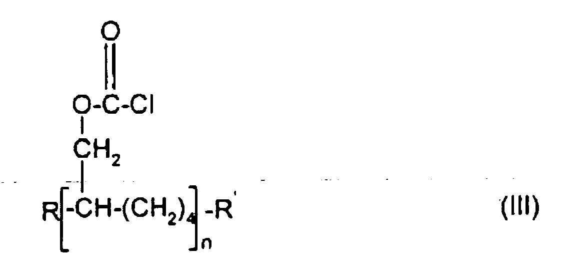 and then converting the polychlorocarbonic ester III into the polycarbamate I using ammonia.

4. The process as claimed in claim 3, wherein the reaction is carried out in an aprotic solvent.

5. The process as claimed in claim 3 or 4, wherein the reaction with phosgene is carried out at +5 to +65°C and the reaction with ammonia is carried out at 5 to 50°C.

6. The use of the polycarbamates of the formula I according to Claim 1 as coating constituents.

7. The use as claimed in claim 6 in automotive finishes.

## Revendications

1. Polycarbamates de formule générale I ou leur mélange d'homologues 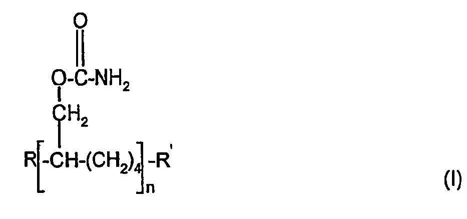 dans laquelle
R et R' sont semblables ou différents et représentent des groupes alkyle ayant 1 à 4 atomes de C et
n est un chiffre entier, la moyenne de n étant un chiffre de 2 à 5.

2. Polycarbamates selon la revendication 1, **caractérisés en ce qu'**ils sont constitués d'un mélange d'homologues avec n = 1 à n = 10.

3. Procédé de préparation de polycarbamates de formule générale I selon la revendication 1 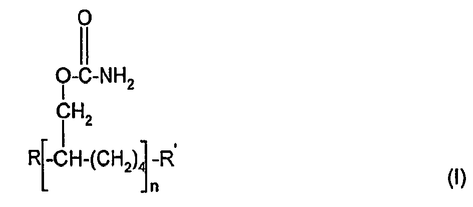 dans laquelle
R et R' sont des groupes alkyle semblables ou différents ayant 1 à 4 atomes de C et
n est un chiffre en moyenne de 2 à 5,
**caractérisé en ce que** l'on fait réagir un polyalcool de formule II 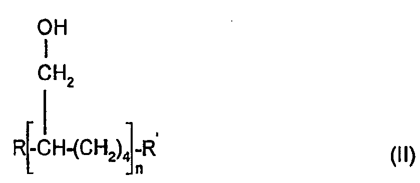 dans laquelle R, R' et n ont la signification précédemment citée,
avec du phosgène pour donner un ester d'acide polychlorocarboxylique de formule III 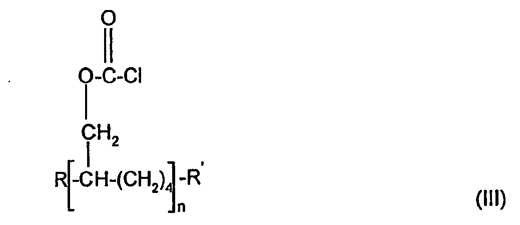 et l'on convertit ensuite l'ester d'acide polychlorocarboxylique III en polycarbamate I avec de l'ammoniac.

4. Procédé selon la revendication 3, **caractérisé en ce que** l'on effectue la réaction dans un solvant aprotique.

5. Procédé selon la revendication 3 ou 4, **caractérisé en ce que** l'on effectue la réaction avec du phosgène à une température de +5 à +65°C et la réaction avec de l'ammoniac à une température de 5 à 50°C.

6. Utilisation des polycarbamates de formule générale I selon la revendication 1 comme composant de peinture.

7. Utilisation selon la revendication 6 dans des peintures automobiles.
